# EUROPÄISCHE PATENTSCHRIFT

(11) **EP 1 337 331 B1**
(45) Veröffentlichungstag und Bekanntmachung des Hinweises auf die Patenterteilung: **10.01.2007**
(21) Anmeldenummer: 01992614.6
(22) Anmeldetag: 10.10.2001
(51) Int. Cl.: C07C 209/72

(54) **VERFAHREN ZUR KATALYTISCHEN HYDRIERUNG VON AROMATISCHEN ODER HETEROAROMATISCHEN AMINEN**
METHOD FOR THE CATALYTIC HYDROGENATION OF AROMATIC OR HETEROAROMATIC AMINES
PROCEDE D'HYDROGENATION CATALYTIQUE D'AMINES AROMATIQUES OU HETEROAROMATIQUES

(30) Priorität: 02.11.2000 DE 10054347
(43) Veröffentlichungstag der Anmeldung: 27.08.2003
(73) Patentinhaber: Degussa AG, 40474 Düsseldorf (DE)
(72) Erfinder: HAAS, Thomas, 60322 Frankfurt (DE); JAEGER, Bernd, 64297 Darmstadt (DE); SAUER, Jörg, 48249 Dülmen (DE); VANHEERTUM, Rudolf, B-2930 Brasschaat (BE)
(86) Internationale Anmeldenummer: PCT/EP2001/012043
(87) Internationale Veröffentlichungsnummer: WO 2002/036260

(56) Entgegenhaltungen:
- EP-A- 0 799 814
- EP-A- 0 813 906
- DE-A- 19 737 190
- US-A- 4 380 680

## Beschreibung

Die Erfindung richtet sich auf ein Verfahren zur katalytischen Hydrierung organischer Verbindungen, insbesondere labiler organischer Verbindungen, aus der Reihe der aromatischen und heteroaromatischen Amine in Gegenwart eines Trägerkatalysators mit einer Ruthenium als Aktivmetall enthaltenden Beschichtung. Unter labilen Verbindungen sind hierbei solche zu verstehen, welche mehr als eine hydrierbare Gruppierung oder neben einer hydrierbaren Gruppierung mindestens eine weitere funktionelle Gruppe aufweisen.

Es ist bekannt, organische Verbindungen mit einer oder mehreren ungesättigten Verbindungen und gegebenenfalls zusätzlichen funktionellen Gruppen unter Verwendung eines edelmetallhaltigen Trägerkatalysators zu hydrieren. Das Hydrierergebnis, insbesondere die Selektivität und Stereoselektivität hängen ausser vom katalytisch wirksamen Edelmetall auch von der Struktur des Trägermaterials ab.

Gemäss US-Patent 4,343,955 lassen sich Alkylphenole in Gegenwart eines Trägerkatalysators auf der Basis von Ruthenium auf einem Aluminiumoxidträger zu Alkylcyclohexanolen mit hohem cis-Anteil hydrieren. Das bei der Herstellung des Trägerkatalysators eingesetzte Aluminiumoxid muss eine ausreichend hohe spezifische Oberfläche aufweisen, insbesondere 100 bis 300 m²/g. Als Nebenprodukt wird durch Dehydratisierung ins gewissem Umfang auch das entsprechende Alkylbenzol gebildet.

Erwünscht ist jedoch in vielen Fällen eine gegenüber diesem Dokument weiter gesteigerte Stereoselektivität bei zusätzlich hoher Standzeit des Katalysators.

Die EP 0 814 098 lehrt ein Verfahren zur Umsetzung verschiedener organischer Verbindungen, darunter aromatischer Verbindungen, in denen mindestens eine Hydroxylgruppe oder eine Aminogruppe am aromatischen Kern gebunden ist, ferner Carbonylverbindungen, Nitrile und mehrfach ungesättigte Polymere in Gegenwart eines geträgerten Rutheniumkatalysators. Hohe Umsätze und Ausbeuten sowie hohe Katalysatorbelastungen und lange Standzeiten werden erreicht, wenn 10 - 50 % des Porenvolumens des Trägers von Makroporen mit einem Porendurchmesser von 50 nm bis 10.000 nm und 50 - 90 % des Porenvolumens des Trägers von Mesoporen mit einem Porendurchmesser im Bereich von 2 - 50 nm gebildet werden. Der Träger, bei welchem es sich um Aktivkohle oder um oxidische oder carbidische Materialien handelt, weist vorzugsweise eine BET-Oberfläche von 50 - 500 m²/g auf. Derartige Katalysatoren weisen zwar eine hohe Hydrieraktivität auf, die Stereoselektivität eines damit durchgeführten Verfahrens ist jedoch, wie sich aus dem Beispiel 3 dieses Dokuments ergibt, gering - bei der Hydrierung von p-tert.-Butylphenol werden trans-4-tert.-Butylcyclohexanol und seine cis-Isomeres im Verhältnis 2 zu 1 gebildet.

Zur Hydrierung von ungesättigten Polymeren eignet sich gemäß US-Patent 5,110,779 ein Trägerkatalysator auf der Basis eines makroporösen Trägermaterials, wie Diatomeenerde, Aluminiumoxid oder Aktivkohle mit einer Beschichtung eines Metalls aus der Gruppe VIII, wie Palladium, Platin und Ruthenium. Wesentliches Kennzeichen des Trägerkatalysators und essentiell für eine gute Olefinhydrierung ist die Porenverteilung, wonach 90 % des Porenvolumens aus Poren mit einem Durchmesser von >1.000 Angström besteht und wobei das Verhältnis der Metalloberfläche zur Trägeroberfläche im Bereich von 0,07 bis 0,75 zu 1 liegt. Die beispielhaft verwendeten Trägermaterialien wiesen dabei folgende BET-Oberfläche auf: Diatomeenerde 2,5 - 3,5 m²/g; Al₂O₃ 10 - 15 m²/g; Aktivkohle 6 - 10 m²/g. Aluminiumoxid und Kieselsäure mit einer BET-Oberfläche im Bereich von 30 - etwa 300 m²/g erwiesen sich demgegenüber bei der Hydrierung olefinischer Polymerer wenig katalytisch aktiv.

EP-A 0 813 906 offenbart ein Verfahren zur Umsetzung von organischen Verbindungen in Gegenwart von Katalysatoren, die Ruthenium als Aktivmetall auf einem Träger umfassen, wobei der Träger einen mittleren Porendurchmesser von mindestens 50 nm und eine BET-Oberfläche von höchstens 30 m²/g aufweist und die Menge des Aktivmetalls 0,01 - 30 Gew.-% des Gesamtgewichts des Katalysators beträgt und wobei das Verhältnis der Oberflächen des Aktivmetalls und des Katalysatorträgers kleiner als 0,05 ist. Als Umsetzung von organischen Verbindungen wird auch die Hydrierung von aromatischen Aminen genannt, das Dokument enthält jedoch keine Lehre zur Stereoselektivität dieser Reaktion. In den Beispielen 5, 6 und 7 wird die Hydrierung von Anilin und Toluylendiamin mit einem Katalysator beschrieben, der 0,5 Gew.-% Ruthenium auf einem makroporösen Aluminiumoxid-Träger mit einer BET-Oberfläche von 10 m²/g aufweist. Beispiel 7 enthält keine Angaben zur Stereoisomerenverteilung des bei der Hydrierung von Toluylendiamin erhaltenen Gemischs an cycloaliphatischen Diamin-Isomeren.

Aufgabe der Erfindung ist es demgemäß, ein weiteres Verfahren zur Hydrierung organischer Verbindungen, insbesondere labiler organischer Verbindungen, aus der Reihe der aromatischen und heteroaromatischen Amine in Gegenwart eines Ruthenium-Trägerkatalysators bereitzustellen, das gegenüber den vorbekannten Verfahren wenigstens in einem Merkmal verbessert ist.

Gemäß einer weiteren Aufgabe der Erfindung sollte die Hydrierung von Verbindungen, welche zu Stereoisomeren führen können, mit höherer Stereoselektivität durchgeführt werden können. Gemäß einer weiteren Aufgabe sollte die Katalysatoraktivität bei der Hydrierung labiler Verbindungen gegenüber den vorbekannten Verfahren erhöht sein, und zusätzlich sollte der Gehalt an Nebenprodukten mit der Standzeit des Katalysators nicht zunehmen.

Die genannten Aufgaben sowie weitere, wie sie sich aus der folgenden Beschreibung ergeben, werden durch das erfindungsgemäße Verfahren sowie den zur Durchführung verwendeten Katalysator gelöst.

Gefunden wurde ein Verfahren zur katalytischen Hydrierung einer organischen Verbindung aus der Reihe der aromatischen und heteroaromatischen Amine in Gegenwart eines Trägerkatalysators mit oxidischen, carbidischen, nitridischen oder silikatischen Trägermaterial und einer Ruthenium als Aktivmetall enthaltenden Beschichtung, wobei das Trägermaterial vor der Beladung mit mindestens einem Aktivmetall eine BET(N₂)-Oberfläche von weniger als 10 m²/g aufweist und Diatomeenerde mit einer BET(N₂)-Oberfläche von größer 2 m²/g ausgeschlossen wird, das dadurch gekennzeichnet ist, dass man einen Trägerkatalysator verwendet, dessen Gehalt an Ruthenium 1,6 bis 3 Gew.-% beträgt und dessen Rutheniumgehalt mindestens 50 Gew.-% der Aktivmetalle ausmacht.

Die Unteransprüche des beanspruchten Verfahrens richten sich auf bevorzugte Ausführungsformen, insbesondere auf die Verwendung eines Katalysators, der als Aktivmetalle mindestens 90 Gew.-%, vorzugsweise mindestens 99 Gew.-% Ruthenium enthält. Der Trägerkatalysator kann als Aktivmetalle außer mindestens 50 Gew.-% Ruthenium ein oder mehrere andere katalytisch aktive Metalle, insbesondere Metalle der 1., 7. und 8. Nebengruppe des Periodensystems enthalten. Bei diesen anderen Metallen handelt es sich insbesondere um Palladium, Platin, Kupfer, Kobalt und Nickel.

Bei den zur Herstellung der erfindungsgemäß zu verwendenden Trägerkatalysatoren einzusetzenden Trägermaterialien handelt es sich um ein oxidisches, nitridisches, carbidisches oder silikatisches Material; ausgenommen sind jedoch Diatomeenerden mit einer spezifischen Oberfläche nach BET(N₂) von >2 m²/g. Geeignete oxidische Materialien sind insbesondere natürlich vorkommende und synthetisch hergestellte Oxide von Aluminium, Silizium, Titan, Zirkon, Magnesium, Zink und Gemische hiervon oder Mischkristalle, wie Perowskite, z.B. Mg Al₂O₄. Besonders geeignete oxidische Materialien sind Aluminiumoxid, Titandioxid, Siliziumdioxid und Zirkoniumdioxid. Unter den silikatischen Trägermaterialien sind insbesondere synthetische Aluminiumsilikate sowie Zirkoniumsilikate zu nennen. Unter den nitridischen Trägermaterialien kommen insbesondere Nitride von Aluminium, Silizium, Titan, Zirkonium, Niob und Tantal infrage sowie Nitride, welche mindestens eines dieser Metalle enthalten und zusätzlich ein weiteres Metall. Einsetzbar sind auch Carbide wie Siliziumcarbid. Zu den oxidischen bzw. silikatischen Trägermaterialien sind auch unterschiedlich zusammengesetzte Glasfritten zu verstehen.

Die Trägermaterialien der erfindungsgemäß zu verwendenden Trägerkatalysatoren weisen erfindungsgemäß eine spezifische Oberfläche, gemessen nach dem BET-Verfahren durch N₂-Adsorption, beispielsweise gemäß DIN 66131, von <10 m²/g insbesondere gleich oder kleiner 5 m²/g und besonders bevorzugt 0,1 bis 2 m²/g auf. Zur Hydrierung, Dehydrierung, Hydrogenolyse und aminierenden Hydrierung wurden im Stand der Technik üblicherweise Trägerkatalysatoren verwendet, deren Trägermaterial eine spezifische Oberfläche im Bereich von 50 - 500 m²/g aufweisen - siehe beispielsweise EP 0 814 098. Der Stand der Technik kennt nur wenige auf die Hydrierung gerichtete Dokumente, wobei rutheniumhaltige oxidische Trägerkatalysatoren, deren Trägermaterial 10 bzw. 2.0 m²/g aufweist, zur Anwendung gelangen - siehe US-Patent 5,110,779. Dieses Dokument richtet sich auf die Hydrierung ganz spezieller Verbindungen, legt mit Ausnahme von Diatomeenerden jedoch nicht nahe, für rutheniumhaltige Trägerkatalysatoren ein Trägermaterial mit einer BET-Oberfläche von <10 m²/g, insbesondere kleiner 5 m²/g und besonders bevorzugt 0,1 bis 2 m²/g zu verwenden.

Trägermaterialien mit der erfindungsgemäßen BET-Oberfläche lassen sich beispielsweise durch bekannte Fällungsverfahren oder flammenpyrolitische Verfahren gewinnen, wobei sich an die Herstellung ein Calcinierungsschritt anschließt - in Abhängigkeit von Calcinierungstemperatur und Calcinierungsdauer lassen sich die gewünschten BET-Oberflächen einstellen. Bei einigen der erfindungsgemäß zu verwendenden Trägermaterialien handelt es sich um natürlich vorkommende Produkte, wie beispielsweise Quarz, Rutil und Zirkon.

Die Beschichtung der vorgenannten Trägermaterialien mit Ruthenium und gegebenenfalls zusätzlich weiteren Metallen erfolgt in an sich bekannter Weise. Besonders geeignet ist die sogenannte "incipient wetness method" (publiziert in Preparation of Catalysts, Delmond, B. Jakobs, P.A., Poncalt, G., Amsterdam Elsevier, 1976, Seite 13). Hierbei wird zunächst die Wasseradsorptionskapazität des Trägermaterials bestimmt. Entsprechend dieser Adsorptionskapazität wird eine wässrige Rutheniumchloridlösung oder eine Lösung, welche außer Rutheniumchlorid auch Verbindungen von anderen hydrieraktiven Metallen enthält, in der für die Beschichtung erforderlichen Konzentration hergestellt.

Anschließend wird der Träger mit dieser Lösung behandelt, wobei die gesamte Menge der Lösung adsorbiert wird. Der beladene Träger wird unter Normaldruck oder vermindertem Druck in einer inerten Gasatmosphäre bei vorzugsweise 20 - 100 °C getrocknet. Schließlich wird der imprägnierte Träger zur Bildung der hydrieraktiven Metalle hydriert, vorzugsweise unter Verwendung von Wasserstoff bei einer Temperatur von 100 - 500 °C über eine Zeitdauer von 20 Minuten bis 24 Stunden. Sofern erwünscht wird der hydrierte Trägerkatalysator ausgewaschen. Die genannte Präparationsmethode führt zu einer feinen Verteilung des Rutheniums auf dem Katalysatorträger, wobei die Kristallitgröße im allgemeinen im Bereich von 1 - 5 nm liegt. Erfindungsgemäß enthält der Trägerkatalysator 1,6 bis 3 Gew.-% Ruthenium, wobei Ruthenium mindestens 50 Gew.-%, vorzugsweise mindestens 90 Gew.-%, insbesondere mindestens 99 Gew.-% der aktiven Metalle ausmacht.

Der erfindungsgemäßen Hydrierung zugängliche organische Verbindungen sind aromatische und heteroaromatische Amine, wie 4,4'-, 2,2'- und 2,4'-Diamino-diphenylmethan und Isomerengemische davon. Die spezielle Struktur des zu verwendenden rutheniumhaltigen Trägerkatalysators macht diesen besonders geeignet zur Hydrierung labiler Verbindungen. Unter labilen Verbindungen werden hierbei solche verstanden, welche außer der zu hydrierenden ungesättigten Gruppierung zusätzlich eine oder mehrere funktionelle Gruppen aufweisen und damit durch Folge- oder Nebenreaktionen die Selektivität der Hydrierung mindern können, so daß außer dem hydrierten Zielprodukt auch ein oder mehrere andere Produkte entstehen. Labil sind die zu hydrierenden organischen Verbindungen insbesondere dann, wenn die funktionelle Gruppe in α-, β- oder γ-Stellung zur ungesättigten Gruppierung stehen.

Die Durchführung der Hydrierung erfolgt in an sich bekannter Weise, indem die zu hydrierende organische Verbindung in Anwesenheit oder Abwesenheit eines Lösungsmittels bei Raumtemperatur oder erhöhter Temperatur unter entsprechendem Wasserstoffdruck in Gegenwart des erfindungsgemäßen Trägerkatalysators umgesetzt wird. Der Katalysator kann hierbei in Form eines Suspensionskatalysators oder eines Festbettkatalysators zur Anwendung gelangen.

Die Festbetthydrierung kann in sogenannter Blasenfahrweise (geflutetes Festbett) oder in Rieselbettfahrweise durchgeführt werden. Die Rieselbettfahrweise, bei welcher ein die organische Verbindung enthaltendes flüssiges Medium über das Katalysatorbett rieselt, und Wasserstoff im Gleich- oder Gegenstrom hierzu strömt, wird insbesondere bei der Hydrierung labiler organischer Verbindungen bevorzugt.

Es wurde gefunden, daß die erfindungsgemäß zu verwendenden Trägerkatalysatoren überraschenderweise zu einer höheren Stereoselektivität der Umsetzung führen als Ruthenium-Trägerkatalysatoren auf der Basis eines Trägermaterials mit BET-Oberfläche von >10 m²/g. Gleichzeitig wurde festgestellt, daß die Katalysatoraktivität erfindungsgemäß zu verwendender Trägerkatalysatoren auch nach langer Betriebsdauer konstant bleibt, so daß auch die Produktzusammensetzung des hydrierten Produkts im wesentlichen konstant bleibt. Unter Einsatz vorbekannter Ru-Trägerkatalysatoren steigt mit zunehmender Betriebsdauer der Anteil an Nebenprodukten an und die Katalysatorstandzeit nimmt ab. Es wird vermutet, daß die aufgezeigten Vorteile darauf zurückzuführen sind, daß infolge der Katalysatorstruktur die zu hydrierende organische Verbindung nur kurzzeitig mit der katalytisch wirksamen Oberfläche in Kontakt steht, da sie nicht in Mesoporen festgehalten wird.

Die Vorteile der Erfindung sind:
❖ die neuen Ru-Trägerkatalysatoren sind zur Hydrierung labiler Verbindungen besonders geeignet;
❖ die Katalysatoren zeichnen sich durch eine hohe Standzeit aus;
❖ die Produktzusamrnensetzung des hydrierten Produkts bleibt auch nach längerer Betriebsdauer im wesentlichen konstant;
❖ in Gegenwart des erfindungsgemäßen Ru-Trägerkatalysators lassen sich mehr funktionelle organische Verbindungen in höherer Stereoselektivität gewinnen.

Die nachfolgenden Beispiele und Vergleichsbeispiele verdeutlichen das erfindungsgemäße Verfahren und die hiermit erzielbaren Vorteile.

### Herstellung des Katalysators:

Es wurde die Wasseraufnahme des Trägers in g H₂O pro 100 g Träger bestimmt.

Für die Beladung von 250 ml Träger wurde RuCl₃ in destilliertem Wasser gelöst. 250 ml wurden in einem Dragierkessel vorgelegt und bei rotierendem Kessel mit der RuCL₃-Lösung begossen.

Der beschichtete Träger wurde 16 Stunden an Luft getrocknet, anschließend im Rohrofen auf 200° aufgeheizt. Anschließend wurde der beschichtete Träger bei 200°C 8 Stunden mit Wasserstoff reduziert. Der reduzierte Ru-Trägerkatalysator wurde dreimal mit jeweils 40 ml destilliertem Wasserchlorid freigewaschen. Die nachfolgende Tabelle zeigt wesentliche Merkmale von zwei erfindungsgemäßen Ru-Trägerkatalysatoren (Beispiel 1 und 2) sowie die Merkmale von zwei nicht erfindungsgemäßen Ru-Trägerkatalysatoren (Vergleichsbeispiele 1 und 2).

| | Träger | BET(N₂) g/m² Träger | Träger d (mm) | Ru-Beladung (Gew.-%) |
|---|---|---|---|---|
| B 1 | α Al₂O₃ | ca. 0,2 | 1,5 | 2,0 |
| B 2 | Rutil | 1 | 1, 5 | 1, 6 |
| VB 1 | γ Al₂O₃ | 230 | 1, 2 | 5 , 0 |
| VB 2 | Rutil/Anatas | 52 | 1 | 1,2 |

| | | | | |
|---|---|---|---|---|
| B = Beispiel; VB = Vergleichsbeispiel d = Durchmesser | | | | |

### Beispiel 3

Hydriert wurde 4,4'-Methylendianilin (= 4,4'-MDA) zu 4,4'-Diamino-dicyclohexylmethan (4,4'-HMDA) mit niedrigem trans-trans-isomeren Anteil.

Die Hydrierung wurde kontinuierlich in einer Rieselbettanlage mit 45 ml Reaktorvolumen durchgeführt. Die Anlage bestand aus einer Flüssigkeitsvorlage, dem Reaktor und einem Flüssigkeitsabscheider. Die Reaktionstemperatur wurde über einen Wärmeträger-Öl-Kreislauf eingestellt. Druck und Wasserstoffstrom wurden elektronisch geregelt. Die 4,4'-MDA enthaltende Lösung mit Methanol als Lösungsmittel wurde dem Wasserstoffstrom mit einer Pumpe zudosiert und das Gemisch am Kopf des Reaktors aufgegeben (Rieselbett-Fahrweise). Nach dem Durchrieseln der Lösung durch den Reaktor wurde das Produkt in regelmäßigen Abständen aus dem Abscheider entnommen. Die Konzentration der Roh-MDA-Lösung betrug in allen Fällen 20 Gew.-%. Das Roh-MDA enthielt 85 Gew.-% 4,4'-MDA. Im Roh-HMDA sind noch 10 bis 20 Gew.-% Oligomere enthalten. Der Reaktordruck betrug in allen Fällen 80 bar, die Flüssigkeitsbelastung LHSV 0,5 h⁻¹. Eingesetzt wurde Ru-Trägerkatalysator gemäß Beispiel 1. Die Reaktionstemperatur betrug 100°C.

Der 4,4'-MDA-Umsatz betrug 94 %, die Ausbeute an 4,4'-HMDA 67 %. Der trans-trans-Anteil war 14 %.

### Vergleichsbeispiel 3

Analog zu Beispiel 1 wurde 4,4'-MDA unter Einsatz des nicht erfindungsgemäßen Ru/Al₂O₃-Trägerkatalysators bei 100°C zu 4,4'-HMDA hydriert. Der Umsatz an 4,4'-MDA war 84 %, die Ausbeute an 4,4'-HMDA 59 %. Es stellte sich ein trans/trans-Anteil von 21 % ein.

## Patentansprüche

1. Verfahren zur katalytischen Hydrierung einer organischen Verbindung aus der Reihe der aromatischen und heteroaromatischen Amine in Gegenwart eines Trägerkatalysators mit oxidischen, carbidischen, nitridischen oder silikatischen Trägermaterial und einer Ruthenium als Aktivmetall enthaltenden Beschichtung, wobei das Trägermaterial vor der Beladung mit mindestens einem Aktivmetall eine BET(N₂)-Oberfläche von weniger als 10 m²/g aufweist und Diatomeenerde mit einer BET(N₂)-Oberfläche von größer 2 m²/g ausgeschlossen wird,
**dadurch gekennzeichnet,**
**dass** man einen Trägerkatalysator verwendet, dessen Gehalt an Ruthenium 1,6 bis 3 Gew.-% beträgt und dessen Rutheniumgehalt mindestens 50 Gew.-% der Aktivmetalle ausmacht.

2. Verfahren nach Anspruch 1,
**dadurch gekennzeichnet,**
**dass** das Ruthenium mindestens 90 Gew.-% der trägergebundenden Aktivmetalle ausmacht.

3. Verfahren nach Anspruch 1,
**dadurch gekennzeichnet,**
**dass** das Ruthenium mindestens 99 Gew.-% der trägergebundenen Aktivmetalle ausmacht.

4. Verfahren nach einem der Ansprüche 1 bis 3,
**dadurch gekennzeichnet,**
**dass** der Trägerkatalysator als Aktivmetalle ausser Ruthenium ein oder mehrere Metalle aus der Reihe der 1., 7. und 8. Nebengruppe des Periodensystems enthält.

5. Verfahren nach einem der Ansprüche 1 bis 4,
**dadurch gekennzeichnet,**
**dass** das Trägermaterial des Trägerkatalysators eine HET(N₂)-Oberfläche von 0,1 bis weniger als 5 m²/g aufweist.

6. Verfahren nach Anspruch 5,
**dadurch gekennzeichnet,**
**dass** das Trägermaterial des Trägerkatalysators eine BET(N₂)-Oberfläche im Bereich von 0,1 bis 2 m²/g aufweist.

7. Verfahren nach einem der Ansprüche 1 bis 6
**dadurch gekennzeichnet,**
**dass** man als aromatisches Amin 4,4'- oder 4,2'-Methylendianilin oder ein Isomerengemisch hiervon hydriert, wobei 4,4'- oder 4,2'-Diamino-dicyclohexylmethan oder ein dieses Produkt enthaltendes Reaktionsgemisch erhalten wird.

## Claims

1. Process for catalytically hydrogenating an organic compound from the group of the aromatic and heteroaromatic amines in the presence of a supported catalyst comprising oxidic, carbidic, nitridic and/or silicatic support material and a coating comprising ruthenium as an active metal, the support material, before the loading with at least one active metal, having a BET(N₂) surface area of less than 10 m²/g and excluding diatomaceous earth having a BET(N₂) surface area of greater than 2 m²/g,
**characterized in that**
a supported catalyst is used whose content of ruthenium is 1.6 to 3% by weight and whose ruthenium content makes up at least 50% by weight of the active metals.

2. Process according to Claim 1,
**characterized in that**
the ruthenium makes up at least 90% by weight of the support-bound active metals.

3. Process according to Claim 1,
**characterized in that**
the ruthenium makes up at least 99% by weight of the support-bound active metals.

4. Process according to one of Claims 1 to 3,
**characterized in that**
the supported catalyst comprises, as active metals, apart from ruthenium, one or more metals from the group of transition element groups 1, 7 and 8 of the periodic table.

5. Process according to one of Claims 1 to 4,
**characterized in that**
the support material of the supported catalyst has a BET(N₂) surface area of 0.1 to less than 5 m²/g.

6. Process according to Claim 5,
**characterized in that**
the support material of the supported catalyst has a BET(N₂) surface area in the range from 0.1 to 2 m²/g.

7. Process according to one of Claims 1 to 6,
**characterized in that**
the aromatic amine hydrogenated is 4,4'- or 4,2'-methylenedianiline or an isomer mixture thereof to obtain 4,4'- or 4,2'-diaminodicyclohexylmethane or a reaction mixture comprising this product.

## Revendications

1. Procédé d'hydrogénation catalytique d'un composé organique de la série des amines aromatiques et hétéroaromatiques, en présence d'un catalyseur supporté, comportant un matériau support de type oxyde, carbure, nitrure ou silicate, et comportant un revêtement contenant du ruthénium en tant que métal actif, le matériau support présentant, avant chargement par au moins un métal actif, une aire BET (N₂) inférieure à 10 m²/g, à l'exception d'une terre de diatomées ayant une aire BET (N₂) supérieure à 2 m²/g, **caractérisé en ce qu'**on utilise un catalyseur supporté dont la teneur en ruthénium est de 1,6 à 3 % en poids, et dont la teneur en ruthénium représente au moins 50 % en poids des métaux actifs.

2. Procédé selon la revendication 1, **caractérisé en ce que** le ruthénium représente au moins 90 % en poids des métaux actifs liés au support.

3. Procédé selon la revendication 1, **caractérisé en ce que** le ruthénium représente au moins 99 % en poids des métaux actifs liés au support.

4. Procédé selon l'une des revendications 1 à 3, **caractérisé en ce que** le catalyseur supporté contient en tant que métaux actifs, outre le ruthénium, un ou plusieurs métaux de la série des métaux du 1er, du 7ème et du 8ème groupes secondaires du Tableau Périodique.

5. Procédé selon l'une des revendications 1 à 4, **caractérisé en ce que** le matériau support du catalyseur supporté présente une aire BET (N₂) de 0,1 à inférieure à 5 m²/g.

6. Procédé selon la revendication 5, **caractérisé en ce que** le matériau support du catalyseur supporté présente une aire BET (N₂) comprise dans la plage de 0,1 à 2 m²/g.

7. Procédé selon l'une des revendications 1 à 6, **caractérisé en ce qu'**on hydrogène, en tant qu'amine aromatique, de la 4,4'- ou de la 4,2'-méthylène-dianiline ou un mélange de leurs isomères, en obtenant du 4,4'- ou du 4,2'-diaminodicyclohexylméthane, ou un mélange réactionnel contenant ce produit.
